# EUROPÄISCHE PATENTANMELDUNG

(11) **EP 0 730 875 A2**
(43) Veröffentlichungstag der Anmeldung: **11.09.1996**
(21) Anmeldenummer: 96101167.3
(22) Anmeldetag: 29.01.1996
(51) Int. Cl.: A61L 27/00, A61L 31/00, A61F 2/00, A61F 2/30, A61F 13/00, B22C 9/04

(54) **Bioverträgliches Produkt und Verfahren zu seiner Herstellung sowie Verwendung des Produktes**

(30) Priorität: 04.03.1995 DE 19507637
(71) Anmelder: Ramalho Ortigao, José Flavio, Dr., 89077 Ulm (DE)
(72) Erfinder: Ramalho Ortigao, José Flavio, Dr., 89077 Ulm (DE)
(74) Vertreter: Becker, Maria, Dipl.-Phys.

(57) **Zusammenfassung**

Die Erfindung betrifft ein bioverträgliches Produkt, ein Verfahren zu seiner Herstellung sowie die Verwendung des Produktes. Das bioverträgliche Produkt, wie Formkörper, Substrat oder dergleichen besteht aus synthetischem Material mit einer Kontaktfläche zur Kontaktierung mit biologischem Material. Derartige Materialien werden vorwiegend im medizinischen Bereich, z. B. bei der Herstellung von Wundauflagen eingesetzt. Zur Verbesserung der Biokompatibilität ist gemäß der Erfindung vorgesehen, daß die Topologie der Kontaktfläche in etwa der Topologie einer Oberfläche eines Materials entspricht, dessen Biokompatibilität höher ist als die des synthetischen Materials.

## Beschreibung

Die Erfindung betrifft ein bioverträgliches Produkt, wie Formkörper, Substrat oder dergleichen, gemäß dem Oberbegriff des Patentanspruchs 1 und ein Verfahren zu dessen Herstellung sowie Verwendung des Produktes.

Aus der DE-OS 41 13 268 ist ein Verfahren zur Herstellung eines Implantates mit einer rauhen strukturierten Oberfläche bekannt, gemäß dem von einem Basismodell, das mit Partikeln aus organischen Materialien versehen ist, eine keramische Abdruckform erstellt wird, in die anschließend geschmolzenes Metall gefüllt wird. Die organischen Partikel können Hirse, Reis oder Hagelzucker sein und sind derart gewählt, daß sie rückstandslos veraschen. Die mit diesem Verfahren erhaltene rauhe Oberfläche dient dazu, daß das Implantat besser an dem an das Implantat anwachsenden Knochen fixiert ist.

Unter bioverträglichen Produkten wird im folgenden ein Produkt aus einem Material verstanden, das mit lebendem Gewebe, lebenden Zellen oder allgemein mit biologischen Materialien verträglich ist. Das Material ist für das lebende Gewebe insbesondere nicht toxisch oder wachstumshemmend.

Wenn die Materialien, aus denen diese Produkte bestehen, bei Kontaktierung mit biologischem Material in einer geeigneten Art und Weise mit dem biologischen Material wechselwirken, spricht man von biokompatiblen Materialien. Die Biokompatibilität ist eine Größe die abhängig ist von der Wechselwirkung des biokompatiblen Materials mit dem biologischen Material, z.B. dem lebenden Gewebe, wobei die Wechselwirkung von molekularen Erkennungsvorgängen abhängt. Die Erkennungsvorgänge umfassen komplexe molekulare Effektor-Rezeptor Wechselwirkungen an den in Kontakt kommenden Oberflächen.

Synthetische Materialien haben im Allgemeinen die Nachteile, daß sie nicht in geeigneter Weise mit den Rezeptoren des biologischen Materials wechselwirken selbst wenn das Material biokompatible Eigenschaften, wie nichttoxische und nichtwachstumshemmende Eigenschaften, aufweist (Y.Ikada, "Interfacial Biocompatibility" in "Polymers of Biological and Biomedical Significance", S.W.Shalaby et al eds., ACS Symposium Series 540, ACS, Washington, 1994, S.35-48).

Biokompatible Materialien finden eine große Anzahl von Anwendungen in der biomedizinischen Industrie. Sie werden beispielsweise eingesetzt als medizinische Implantate oder auch bei der Herstellung von Behältern und Substraten für Zellkulturen, beispielsweise differenzierte Zellen, wie Keratinozyte oder Chondrozyte.

Um die Biokompatibilität von synthetischen Materialien zu verbessern wurden entweder Biomoleküle, wie beispielsweise Kollagen, auf die Oberfläche des synthetischen Materials aufgepfropft oder die Oberfläche chemisch modifiziert durch Aufbringen von funktionellen Gruppen oder Aufpfropfen von Polymerketten (vgl. I.V.Yannas, "Regeneration of skin and nerve by use of collagen templates. Collagen III", M.E.Nimni ed., CRC Press, 1988, Boca Raron, Fl. und R.Barbucci, M.Benvenuti, F.Tempesti, in "Polymeric Biomaterials", Marcel Dekker, New York 1994, S.199-230).

Das Aufpfropfen von Biomolekülen wie Kollagen auf die synthetische Oberfläche hat die Nachteile, daß die Sterilisation dieses Gebildes stark beeinträchtigt ist und das Gebilde nur sehr kurzlebig ist aufgrund der Selbstzerstörung der Biomoleküle. So produzieren Zellen beispielsweise Protease, die Kollagen abbauen. Ein weiterer Nachteil ist, daß die Biomoleküle nur schlecht zu beschaffen und schwierig zu bearbeiten und handzuhaben sind.

Die chemische Modifikation der Oberfläche des synthetischen Materials ist nachteilig, da die biologischen Oberflächen komplexer Natur sind und die Mechanismen der molekularen Erkennung nur wenig verstanden sind. Deshalb ist es schwierig die räumliche Anordnung der funktionellen Grupppen zu bestimmen um eine gute Wechselwirkung mit dem biologischen Rezeptoren zu erhalten.

Weiter wurde in Zusammenhang mit der Erforschung von molekularer Erkennung ein Verfahren entwickelt, bei dem funktionelle Monomere um ein "Präge"-Molekül herum polymerisieren (K.Mosbach, "Molecular Imprinting" in Trends in Biochemical Science 19, Seite 9-14). Das Präge-Molekül wird dann von dem Polymer entfernt und es bleibt ein freier Platz in den das Präge-Molekül passt. Das Polymer kann dann später das Molekül "erkennen", da nur das Präge-Molekül in den freien Platz paßt. Derartige Polymere könnten in der Zukunft Anwendung finden als maßgeschneiderte Stoffe zum Abtrennen von Molekülen, als katalytisch wirksame Polymere für die organische Synthese oder als Sensoren. Diese Anwendungen sind aber noch nicht durchführbar, denn beispielsweise wirft die Größenordnung der Präge-Moleküle von unter 1 nm Probleme auf. Für die katalytische Anwendung besteht insbesondere das Problem, daß eine Prägung eines Zwischenzustandes des Präge-Moleküls, der aber sehr kurzlebig ist, in das Polymer eingebracht werden müsste.

Ausgehend von diesem Stand der Technik ist es Aufgabe der Erfindung ein Produkt bereitzustellen, das eine verbesserte Biokompatibilität aufweist und einfach und wirtschaftlich herzustellen und handzuhaben ist.

Diese Aufgabe wird gelöst durch ein Produkt mit den Merkmalen des Anspruchs 1.

Wenn die Oberfläche des bioverträglichen Produktes, die eine Kontaktfläche mit biologischem Material bilden soll, eine Topologie aufweist, die in etwa der Geometrie der Oberfläche und Spatialanordnung von chemischen Gruppen einer Oberfläche eines Materials entspricht, dessen Biokompatibilität höher ist als die des synthetischen Materials, erhöht sich die Biokompatibilität des synthetischen Materials überraschenderweise erheblich gegenüber der Biokompatibilität eines synthetischen Materials mit einer "glatten" Oberfläche. Auf diese Weise wird ein biokompatibles Produkt erhalten, das einfach aufgebaut ist, da es nur aus einem einzigen synthetischen Material besteht und zudem günstig und einfach herzustellen ist. Das Material kann ein dünner Film, also eine Schicht oder ein Formkörper sein.

Die erhöhte Biokompatibilität könnte darauf zurückgeführt werden, daß überraschenderweise die Grobstruktur und nicht die molekulare Feinstruktur der Oberfläche zu einem wesentlichen Teil ausschlaggebend dafür ist, daß sich das Protein des biologischen Materials in geeigneter Weise falten kann. Ein weiterer Grund könnte darin bestehen, daß die Biokompatibilität an der Grenzfläche zwischen synthetischem und biologischem Material auch von makroskopischen Komponenten abhängt und daß die Rezeptoren und Liganden durch Erhöhungen und Vertiefungen der geometrischen Oberfläche, also der Toplogie, nachgeahmt werden können.

Die Topologie der Oberfläche des Materials höherer Biokompatibilität läßt sich am einfachsten in Form eines positiven Abdrucks, gemäß Anspruch 2, auf die Kontakfläche übertragen.

Um eine gute Biokompatibilität zu erreichen, liegen die topologischen Strukturen der Kontaktfläche im Bereich von 10 bis 300 nm, vorzugsweise 10 bis 100 nm und am bevorzugsten zwischen 10 bis 30 nm.

Als synthetische Materialien für das bioverträgliche Produkt können je nach dem Anwendungszweck verschiedene Materialien zum Einsatz kommen, beispielsweise ein polymeres Material, Silikon, Siloxan, Methylmetacrylat oder auch ein Metall.

Das Material höherer Biokompatibilität ist vorzugsweise Kollagen, Basaltamina, Heparin, Dextran oder ein organisches Gewebe.

In weiteren Ausgestaltungen weist die Kontaktfläche gemäß Anspruch 13 und 14 eine Ladungsverteilung und/oder eine chemische Struktur auf. Eine geeignete Ladungsverteilung bzw. eine chemische Struktur, also die gezielte Anbringung von Ladungen bzw. funktionellen, molekularen Gruppen auf der Kontaktfläche, erhöht die Biokompatibilität weiter.

Im folgenden wird die Erfindung anhand eines Beispiels im einzelnen beschrieben.

Das bioverträgliche Produkt dieses Ausführungsbeispiels wurde in einem Herstellungsverfahren mit den folgenden Verfahrensschritten hergestellt:
1. Als Material mit einer höheren Biokompatibilität wurde Kollagen, das im Handel als Pulver erhältlich ist, verwendet. Dieses wurde in Wasser in einer Konzentration von 1% bei einem pH-Wert von 4 aufgelöst. 0,05 bis 0,2 ml dieser Lösung wurden auf einem Objektträger geträufelt und zu einem sehr dünnen Film getrocknet. Verschiedene Techniken zum Herstellen einer Kollagen-Matrix sind bei Y.Kuroyanagi et al., Ann. Plastic Surgery,31 (1993) S.340-351 beschrieben. Nach der Fertigstellung wurde die Kollagen-Matrix elektronenmikroskopisch qualitativ untersucht.
2. Die Matrix wurde in Kontakt gebracht mit einem Abdruckmaterial. Das Abdruckmaterial sollte die folgenden Eigenschaften aufweisen:
   - Es muß die Oberflächentopologie des biologischen Materials in einer Größenordnung von 10 bis bis 300 nm, vorzugsweise 10 bis 100 nm wiedergeben können. Besonders bevorzugt wird Material, das die topologischen Strukturen in der Größenordnung von 10 bis 30 nm wiedergeben kann.
   - Es muß unter physiologischen Bedingungen polymerisieren.
   - Es darf die Struktur der Oberfläche des biologischen Materials während der Polymerisation nicht verändern.
   - Es darf nicht mit dem synthetischen Material des herzustellenden Produktes reagieren.

   Als geeignete Materialien erwiesen sich photopolymerisierbare Siloxane und Methylmetacrylate. Besonders gute Resultate wurden mit einem Methylmetacrylat erzielt, das unter der Marke "Permadyne" erhältlich ist.
   Ein Gramm des Abdruckmaterials wurde mit einem Polymerisationsstarter in einem Verhältnis von 1:5 bis 1:30, vorzugsweise 1:15 bis 1:20 gemischt. Diese Mischung wurde auf einen Objektträger aufgebracht und zu einer dünnen Schicht ausgewalzt und dann mit der Matrix in Kontakt gebracht.
3. Die mit der Matrix in Kontakt stehende Schicht Abdruckmaterial konnte innerhalb drei Stunden bei Zimmertemperatur kontaktpolymerisieren.
4. Das polymerisierte Abdruckmaterial wurde von der Matrix getrennt. Damit weist das Abdruckmaterial auf seiner Oberfläche einen negativen Abdruck der Oberfläche der Kollagenmatrix auf.
5. Von diesem negativen Abdruck wurde ein weiterer Abdruck gefertigt aus einem synthetischen Material. Dieser weitere Abdruck ist somit ein positiver Abdruck der Oberfläche der Kollagenmatrix.
   Als synthetische Materialien wurden photopolymerisierbare Siloxane und Methylmetacrylate sowie Materialien, die im Handel unter den Bezeichnungen "Technovit" und "Araldit" erhältlich sind, eingesetzt.

Dieser aus den genannten Materialien bestehende, positive Abdruck der Kollagenmatrix stellt dann das bioverträgliche Produkt dar, dessen Oberfläche eine Topologie aufweist, die in etwa der Topologie der Kollagenmatrix entspricht und als Kontaktfläche zur Kontaktierung mit biologischem Material dient.

Obwohl eine strenge Definition der Biokompatibilität nicht möglich ist, ist es allgemein anerkannt, daß ein charakteristisches Merkmal der Biokompatibilität eines Produktes die Fähigkeit ist, das Wachstum von differenzierten Zellen, wie Keratinocyten, zu unterstützen.

Um zu zeigen, daß die Biokompatibilität des erfindungsgemäßen Produktes größer ist als die Biokompatibilität des synthetischen Materials mit einer nicht erfindungsgemäß strukturierten Kontaktfläche wurde der folgende Versuch durchgeführt.

Es wurden zwei Ausführungen des Produktes miteinander verglichen. Die erste war ein Film mit einer erfindungsgemäßen Kontaktfläche und die zweite ein Film mit einer unbehandelten Kontaktfläche. Die Filme wurden in einem Autoklaven bei 121°C und 1,1 atü für 25 min sterilisiert. Die sterilen Filme wurden mit einer Salzlösung gewaschen, in ein Gefäß eingebracht und in mit 10% FBS versetztes DMEM eingetaucht. Dann wurden auf die Filme jeweils 1,3*10⁵ Zellen aufgebracht. Die Zellen konnten sich über 12 Stunden bei 37°C in einer Atmosphäre mit 5% CO₂ proliferieren oder wuchten. Danach wurden die Filme unter dem Mikroskop untersucht und die Dichte der Zellen mit einer an das Mikroskop angebrachten Videokamera aufgezeichnet.

Es zeigte sich eine erheblich gesteigerte Zellenvermehrung auf den Filmen, deren Kontaktfläche die erfindungsgemäße Ausgestaltung aufwiesen.

Anstelle einer Kollagenmatrix wurde in einem weiteren Ausführungsbeispiel eine Matrix aus menschlicher Haut gefertigt, indem kleine Stücke der Haut mit einer Mischung von 2,5% Glutaraldehyd und 2% Formaldehyd in 0,067 M Cacodylat Puffer (pH 7,4) für 30 min bei Zimmertemperatur fixiert wurden. Diese Stücke wurden 1 mm³ große Proben geschnitten und für 30 min nochmal fixiert. Nach Spülen der Proben mit 0,1 M Cacodylat Puffer (pH 7,4) wurden die Proben in flüssigem Stickstoff gefroren.

Die erfindungsgemäßen bioverträglichen Produkte mit erhöhter Biokompatibilität finden vielseitige Anwendungen.

So können sie beispielsweise als Implantate oder als mit dem lebenden Gewebe in Kontakt kommende Oberflächen von Implantaten dienen, damit die Implantate von dem Körper besser angenommen zu werden. Wenn das Implantat beispielsweise in einem Knochen eingesetzt werden soll, wäre es dann vorteilhaft bei der Herstellung des bioverträglichen Produkts als Material höherer Biokompatibilität einen Knochen zu nehmen.

Eine weitere Anwendungsmöglichkeit bietet sich bei der Herstellung von Substraten aus synthetischen Materialien für die Züchtung von Zellkulturen. Substrate mit der erfindungsgemäßen Ausgestaltung der Kontaktfläche sind aufgrund ihrer gesteigerten Biokompatibilität und damit einhergehenden Unterstützung der Zellvermehrung äußerst vorteilhaft einsetzbar.

Auch bei der Herstellung von Wundauflagen kann das erfindungsgemäße Produkt vorteilhaft eingesetzt werden, denn die Wunde kann schneller heilen, wenn der mit dem lebenden Gewebe in Kontakt kommende Teil der Wundauflage eine hohe Biokompatibilität aufweist. In diesem Fall käme bei der Herstellung des erfindungsgemäßen Produktes als Material höherer Biokompatibilität z.B. die Haut in Betracht.

## Patentansprüche

1. Bioverträgliches Produkt, wie Formkörper, Substrat oder dergleichen, aus synthetischem Material mit einer Kontaktfläche zur Kontaktierung mit biologischem Material,
**dadurch gekennzeichnet, daß**
die Topologie der Kontaktfläche in etwa der Topologie einer Oberfläche eines Materials entspricht, dessen Biokompatibilität höher ist als die des synthetischen Materials.

2. Bioverträgliches Produkt nach Anspruch 1, dadurch gekennzeichnet, daß die Kontaktfläche des synthetischen Materials ein positiver Abdruck der Oberfläche des Materials höherer Biokompatibilität ist.

3. Bioverträgliches Produkt nach Anspruch 1 oder 2, dadurch gekennzeichnet, daß die topologischen Strukturen der Kontaktfläche des synthetischen Materials in der Größenordnung von 10 bis 300 nm liegen.

4. Bioverträgliches Produkt nach Anspruch 1 oder 2, dadurch gekennzeichnet, daß die topologischen Strukturen der Kontaktfläche des synthetischen Materials in der Größenordnung von 10 bis 100 nm liegen.

5. Bioverträgliches Produkt nach Anspruch 1 oder 2, dadurch gekennzeichnet, daß die topologischen Strukturen der Kontaktfläche des synthetischen Materials in der Größenordnung von 10 bis 30 nm liegen.

6. Bioverträgliches Produkt nach einem der vorhergehenden Ansprüche, dadurch gekennzeichnet, daß das synthetische Material ein polymeres Material ist.

7. Bioverträgliches Produkt nach einem der Ansprüche 1 bis 5, dadurch gekennzeichnet, daß das synthetische Material ein Silikon ist.

8. Bioverträgliches Produkt nach einem der Ansprüche 1 bis 5, dadurch gekennzeichnet, daß das synthetische Material Siloxan ist.

9. Bioverträgliches Produkt nach einem der Ansprüche 1 bis 5, dadurch gekennzeichnet, daß das synthetische Material Methylmetacrylat ist.

10. Bioverträgliches Produkt nach einem der Ansprüche 1 bis 5, dadurch gekennzeichnet, daß das synthetische Material ein Metall ist.

11. Bioverträgliches Produkt nach einem der vorhergehenden Ansprüche, dadurch gekennzeichnet, daß das Material höherer Biokompatibilität Kollagen, Heparin oder Dextran ist.

12. Bioverträgliches Produkt nach einem der vorhergehenden Ansprüche, dadurch gekennzeichnet, daß das Material höherer Biokompatibilität ein organisches Gewebe ist.

13. Bioverträgliches Produkt nach einem der vorhergehenden Ansprüche, dadurch gekennzeichnet, daß die Kontaktfläche des bioverträglichen Produkts eine Ladungsverteilung aufweist.

14. Bioverträgliches Produkt nach einem der vorhergehenden Ansprüche, dadurch gekennzeichnet, daß die Kontaktfläche des bioverträglichen Produkts eine chemische Struktur aufweist.

15. Verfahren zur Herstellung eines bioverträglichen Produkts nach einem der vorhergehenden Ansprüche, gekennzeichnet durch folgende Schritte:
- Herstellen einer Matrix aus dem Material höherer Biokompatibilität,
- Kontaktieren eines polymerisierbaren Abdruckmaterials mit der Matrix,
- Kontaktpolymerisation des polymerisier-baren Abdruckmaterials,
- Trennen der Matrix von dem polymerisierten Abdruckmaterial und
- Fertigen des bioverträglichen Produkts in Form eines Abdrucks von der mit der Matrix kontaktierten Oberfläche des Abdruckmaterials aus einem synthetischen Material.

16. Verfahren nach Anspruch 15, dadurch gekennzeichnet, daß die Matrix aus Kollagen besteht.

17. Verfahren nach Anspruch 15, dadurch gekennzeichnet, daß das Abdruckmaterial photopolymerisierbares Siloxan oder Methylmetacrylat ist.

18. Verwendung des bioverträglichen Produkts nach einem der vorhergehenden Ansprüche 1 bis 14 als Implantat.

19. Verwendung des bioverträglichen Produkts nach einem der vorhergehenden Ansprüche 1 bis 14 als der mit dem lebendem Gewebe in Kontakt kommende Teil eines Implantats.

20. Verwendung des bioverträglichen Produkts nach einem der vorhergehenden Ansprüche 1 bis 14 als Substrat für das Züchten von Zellkulturen.

21. Verwendung des bioverträglichen Produkts nach einem der vorhergehenden Ansprüche 1 bis 14 als der mit der Wunde und/oder der Haut in Kontakt kommende Teil einer Wundauflage.

22. Verwendung des bioverträglichen Produkts nach einem der vorhergehenden Ansprüche 1 bis 14 als Wundauflage.
